# EUROPÄISCHE PATENTSCHRIFT

(11) **EP 4 566 659 B1**
(45) Veröffentlichungstag und Bekanntmachung des Hinweises auf die Patenterteilung: **04.03.2026**
(21) Anmeldenummer: 24217990.1
(22) Anmeldetag: 06.12.2024
(51) Int. Cl.: A61N 1/44

(54) **VORRICHTUNG ZUR BEHANDLUNG VON BANDSCHEIBENVORFÄLLEN DURCH KALTES ATMOSPHÄRISCHES PLASMA**
APPARATUS FOR TREATING INTERVERTEBRAL DISC INCIDENTS BY COLD ATMOSPHERIC PLASMA
DISPOSITIF DE TRAITEMENT D'ACCIDENTS DE DISQUE INTERVERTEBRAL PAR PLASMA ATMOSPHERIQUE FROID

(30) Priorität: 08.12.2023 DE 102023212411
(43) Veröffentlichungstag der Anmeldung: 11.06.2025
(73) Patentinhaber: NINURTA GmbH, 75382 Althengstett (DE)
(72) Erfinder: Al-Hamdani, Yahya, 75382 Althengstett (DE)
(74) Vertreter: Kohler Schmid Möbus Patentanwälte

(56) Entgegenhaltungen:
- WO-A1-2018/230789
- WO-A1-2023/078913
- KR-B1- 101 422 823
- US-A1- 2013 072 859
- US-A1- 2015 094 647
- US-A1- 2016 331 439
- US-A1- 2019 327 823
- US-A1- 2023 142 077
- US-A1- 2023 292 413
- ANONYMOUS SERGEY ET AL: "COLD PLASMA NUCLEOPLASTY FOR INTERVERTEBRAL DISC HERNIATION | Kolesov | Russian Journal of Spine Surgery (Khirurgiya Pozvonochnika)", no. 3, 27 September 2007 (2007-09-27), pages 53 - 58, XP093265848, ISSN: 1810-8997, Retrieved from the Internet <URL:https://www.spinesurgery.ru/jour/article/view/932?locale=en_US> DOI: 10.14531/ss2007.3.53-58

## Beschreibung

### Hintergrund der Erfindung

Die Erfindung betrifft eine Vorrichtung zur Behandlung von Bandscheibenvorfällen, insbesondere im Veterinärwesen, vorzugsweise bei Hunden und Katzen. Es kommt dabei ein Gerät zur Erzeugung kalten atmosphärischen Plasmas zum Einsatz, welches eine Elektrode und ein die Elektrode zumindest teilweise umgebendes plattenförmiges Dielektrikum aufweist. Die Erfindung betrifft auch kaltes atmosphärisches Plasma im Rahmen einer weiteren medizinischen Indikation.

Bandscheibenvorfälle ereignen sich häufig aufgrund verschiedenartiger degenerativer Prozesse im Körper. Bei tierischen Patienten, insbesondere Hunden, in höherem Alter können sie oftmals körperlichen Veränderungen zugeschrieben werden, welche aufgrund eines normalen senilen Umbaus und/oder einer rassentypischen Prädisposition stattfinden. Dennoch werden degenerative Prozesse, die zu einem Bandscheibenvorfall führen können, auch bei jüngeren tierischen Patienten, insbesondere Hunden, beobachtet. Bei diesen jüngeren Patienten kann eine Erkrankung oder Verletzung der Bandscheibe vorliegen. Bekannte Degenerationsprozesse, welche zu Bandscheibenvorfällen bei Hunden führen können, lassen sich in chondroide und fibroide Degenerationsprozesse unterscheiden. Das Behandlungsverfahren, welches unter Ausnutzung der Erfindung angestrebt wird, richtet sich vornehmlich, aber nicht ausschließlich, auf die Behandlung chronischer Bandscheibenvorfälle, welche aufgrund von fibroider Degeneration, insbesondere bei Hunden, auftreten. Bisherig etablierte Verfahren nutzen zu diesem Zweck entweder einen konservativen Behandlungsansatz bestehend aus Schmerzkontrolle, Entzündungshemmung und Physiotherapie oder erfordern einen chirurgischen Eingriff.

Als Plasma wird ein ionisiertes Gas oder Gasgemisch bezeichnet. Neben neutralen Gasteilchen treten dabei Ionen, angeregte Atome oder Moleküle sowie freie Elektronen auf. Im Rahmen spontaner Emission kann durch die angeregten Teilchen sichtbares Licht und/oder UV-Strahlung ausgesendet werden.

Kaltes atmosphärisches Plasma für medizinische Anwendungen wird typischerweise aus Luft bei Atmosphärendruck unter Aufwendung elektrischer Energie generiert. Die Beschaffenheit des Plasmas wird dabei unter anderem von der Umgebungstemperatur, der Stärke des anliegenden elektrischen Feldes sowie der Entladungsgeometrie beeinflusst. Die erste medizinische Indikation kalten atmosphärischen Plasmas leitet sich insbesondere aus dessen Fähigkeit ab, bei sachgemäßer Anwendung Bakterien, Viren und Pilze abzutöten, ohne eine nennenswerte Schädigung der behandelten Körperoberfläche hervorzurufen. Weiterhin hat eine Plasmaanwendung das Potential, die Mikrozirkulation innerhalb des Hautgewebes zu fördern, was wiederum zu einer verbesserten Sauerstoffsättigung des Gewebes beiträgt. Dadurch wird unter anderem die Kollagensynthese erhöht, die Zellmigration optimiert sowie die Vermehrung von bei der Gewebereparatur beteiligter Zellen gefördert. In diesem Zusammenhang wird kaltes atmosphärisches Plasma bisher vornehmlich zur Behandlung von Hautkrankheiten oder Wundheilungsstörungen, sowohl im humanmedizinischen als auch im veterinärmedizinischen Bereich, erfolgreich eingesetzt. Die aufgeführten Wirkungsmechanismen bedingen zusätzlich auch Anwendungsmöglichkeiten rein kosmetischer Natur.

Geräte zur Erzeugung kalten atmosphärischen Plasmas können über das Prinzip der dielektrisch behinderten Entladung (auch dielektrische Barriereentladung genannt) betrieben werden. Eine Elektrode ist dabei über ein elektrisch isolierendes Material, das Dielektrikum, von einem Gasraum, in welchem das Plasma erzeugt werden soll, getrennt. Über die Elektrode wird mithilfe hochfrequenter elektrischer Schwingungen ein elektrisches Feld erzeugt. In dem Gasraum zwischen dem Dielektrikum und dem behandelten Körper können in der Folge in begrenztem Maße Entladungsvorgänge zur Plasmaerzeugung stattfinden. Die Begrenzung der Entladungen erfolgt durch die Ansammlung von Ladungsträgern auf der Oberfläche des Dielektrikums unter Ausbildung eines elektrischen Gegenfeldes. Bei dem Vorgang bilden sich somit vorzugsweise mehrere Plasmafilamente begrenzter Lebensdauer aus.

Geräte dieser Art weisen für gewöhnlich eine Kontrolleinheit auf, in welcher unter anderem ein Resonanzwandler zur effizienten Erzeugung der elektrischen Hochfrequenzschwingungen aus einem zur Verfügung stehenden elektrischen Netzanschluss verbaut ist. Weiterhin befindet sich die Elektrode und das daran angeordnete Dielektrikum oftmals in einem Handstück, um eine bequeme und zweckmäßige Applikation des Plasmas zu ermöglichen. Die Stärke des angelegten elektrischen Feldes kann innerhalb gewisser Grenzen an der Kontrolleinheit vorgegeben werden.

Das Dielektrikum liegt dabei in Form einer flachen Platte oder Scheibe vor. Das Material und die Wandstärke des Dielektrikums sind in der Regel so angepasst, dass unkontrollierte Entladungen verhindert werden und ein Plasma mit einer Beschaffenheit erzeugt werden kann, welche sich zur Behandlung der Hautoberfläche und eventuell der subkutanen Schichten geringer Tiefe eignet.

WO 2018/230 789 A1 beschreibt eine Vorrichtung zum Erzeugen kalten atmosphärischen Plasmas mit einer Elektrode mit einem plattenförmigen Dielektrikum. Die Vorrichtung bzw. das mittels der Vorrichtung erzeugte Plasma eignet sich insbesondere zur Hautbehandlung.

US 2023/0292413 A1 beschreibt unter anderem eine Methode zur Plasmaerzeugung, wobei ein mit einem metallischen Film beschichtetes Dielektrikum zum Einsatz kommt. Weiterhin wird die Möglichkeit der Injektion von Polyethylenglycol, insbesondere als Trägersubstanz für weitere pharmazeutisch wirksame Substanzen oder Substanzen zur Energiemodulation, zur Unterstützung von Behandlungsmethoden eines menschlichen oder tierischen Körpers dargestellt.

Kolesov, S.V. und Kurpyakov, A.P. beschreiben in "Cold Plasma Nucleoplasty for Intervertebral Disc Herniation" (Russian Journal of Spine Surgery (Khirurgiya Pozvonochnika). 2007;(3):053-058) ein Nukleoplastieverfahren zur Behandlung von Patienten mit Bandscheibenvorfällen oder degenerativen Bandscheibenerkrankungen. Dabei wird kaltes Plasma verwendet, um gezielt Gewebe aus dem Inneren einer Bandscheibe zu entfernen und so den Druck auf umliegende Nerven zu verringern. Es handelt sich dabei im Gegensatz zur vorliegend beschriebenen Erfindung um ein invasives Verfahren.

Weitere Vorrichtungen zur Plasmaerzeugung und/oder Methoden der Plasmanutzung bei medizinischen oder kosmetischen Behandlungen sind beispielsweise in US 2015/0 094 647 A1, KR 10 14 22 823 B1, US 2013/0 072 859 A1, US 2019/0 327 823 A1, WO 2023/078 913 A1, US 2023/0 142 077 A1 und US 2016/0 331 439 A1 beschrieben.

### Aufgabe der Erfindung

Der Erfindung liegt demgegenüber die Aufgabe zugrunde, eine Vorrichtung und einen Stoff zur Behandlung von Bandscheibenvorfällen, insbesondere von Bandscheibenvorfällen bei Hunden und Katzen, zu schaffen.

### Beschreibung der Erfindung

Diese Aufgabe wird erfindungsgemäß gelöst durch eine Vorrichtung nach Anspruch 1, welche ein Gerät zur Erzeugung kalten atmosphärischen Plasmas mit einer Elektrode und ein die Elektrode zumindest teilweise umgebendes plattenförmiges Dielektrikum aufweist, wobei das Dielektrikum eine Wandstärke t von minimal 1,2 mm und maximal 1,73 mm aufweist und wobei das Dielektrikum einen Eingriffsbereich mit einer Höhe (bzw. Tiefe) im Bereich von minimal 0,1 mm und maximal 0,4 mm aufweist. Bevorzugte Weiterbildungen der Vorrichtung werden von den abhängigen Ansprüchen wiedergegeben.

Bestehende Vorrichtungen zur humanmedizinischen sowie veterinärmedizinischen Nutzbarmachung kalten atmosphärischen Plasmas sind durch eine Begrenzung des anlegbaren elektrischen Feldes im Zusammenspiel mit dem Material und der gewählten Wandstärke des Dielektrikums auf eine Erzielung von Behandlungserfolgen im Zusammenhang mit Hautkrankheiten und Wundheilungsstörungen ausgelegt. Durch die Änderung der Wandstärke des Dielektrikums, insbesondere eine Reduktion der Wandstärke im richtigen Maße, ist es mithilfe der Erfindung möglich, eine gezielte Tiefenwirkung im Wirbelsäulenbereich eines behandelten Körpers hervorzurufen und darüber eine Verbesserung bzw. eine zumindest zeitweise Genesung eines Bandscheibenvorfalles zu erzielen. Durch eine, einem jeweiligen Material und eines anliegenden elektrischen Energieniveaus angepasste Auswahl der Wandstärke der Dielektrikumsplatte, lässt sich das entstehende elektrische Feld sowie die Beschaffenheit des entstehenden kalten atmosphärischen Plasmas zur Erzielung der Tiefenwirkung auslegen. Die Behandlungserfolge sind insbesondere über empirische Studien belegt.

Aus hygienischen Gründen sind Dielektrikumsplatten für gewöhnlich abnehmbar an plasmaerzeugenden Geräten angeordnet. Es ist daher ein besonderer Vorteil der Erfindung, dass erfindungsgemäße Dielektrikumsplatten in der Regel an bestehenden Geräten einfach gegen deren Standarddielektrikumsplatten ausgetauscht und bei Bedarf auch wieder rückgetauscht werden können.

In einer bevorzugten Ausführung der Erfindung weist die Dielektrikumsplatte Kunststoff auf, insbesondere ein Acrylnitril-Butadien-Styrol-Copolymer. Vorteile eines Dielektrikums auf Kunststoffbasis sind aufgrund einer kostengünstigen Herstellung sowie aufgrund der Recyclingfähigkeit gegeben. Die vorgesehene Wandstärke kann bei der Herstellung in präzisem Maße eingehalten werden. Insbesondere ist auch eine Herstellung über effiziente additive Fertigungsverfahren möglich.

Bei einer Ausführungsvariante der Erfindung weist das Dielektrikum einen keramischen Werkstoff auf. Die elektrisch isolierenden Eigenschaften gewisser keramischer Werkstoffe können analog derer von Kunststoff zur Herstellung eines Dielektrikums genutzt werden. Die Wandstärke des Dielektrikums auf Keramikbasis muss dabei eventuell geringfügig gegenüber der Wandstärke eines entsprechenden Dielektrikums auf Kunststoffbasis angepasst werden.

In einer vorteilhaften Ausführung der Erfindung liegt die Dielektrikumsplatte in Form einer flachen Scheibe vor, wobei die Scheibe vorzugsweise einen Durchmesser zwischen 14 mm und 51 mm aufweist. Dielektrikumsscheiben dieser Art können insbesondere bei plasmaerzeugenden Geräten, welche für kosmetische Anwendungen konzipiert wurden (oftmals als Plasmapen bezeichnet), eingesetzt werden.

Dem Eingriffsbereich kann die Aufgabe zukommen die Dielektrikumsscheibe im Zusammenspiel mit einer Feder oder einem Vorsprung an einem Handstück des plasmaerzeugenden Geräts gegen Verschiebungen zu sichern. Es ist denkbar, die Feder oder den Vorsprung so zu gestalten, dass diese als Teile der Elektrode fungieren. Weiterhin ist die Möglichkeit gegeben, die Dielektrikumsscheibe schon während der Herstellung über das Einbringen einer metallischen Komponente in den Eingriffsbereich auf eine bestimmte Elektrode anzupassen.

Der Eingriffsbereich einer Dielektrikumsscheibe ist vorzugsweise ebenfalls kreisrund gestaltet und weist einen deutlich geringeren Durchmesser d_{E} als die Scheibe selbst auf. Der Durchmesser d_{E} des Eingriffsbereichs kann vorzugsweise zwischen 2 mm und 3 mm liegen.

Bei einer bevorzugten Ausführung der Erfindung wird die notwendige elektrische Energie über einen gewöhnlichen Hausnetzanschluss, welcher eine Wechselspannung mit 100 V - 240 V in einer Frequenz von 50 Hz oder 60 Hz zur Verfügung stellt, bezogen. Zur Erzeugung des kalten atmosphärischen Plasmas oftmals genutzte elektrische Hochfrequenzschwingungen im kHz-Bereich werden wiederum bevorzugt über einen Resonanzwandler in effizienter Art und Weise erzeugt.

Bei einer weiteren bevorzugten Ausführungsvariante der Erfindung weist die Vorrichtung ein Injektionsgerät zur Injektion von Polyethylenglycol auf. Insbesondere kann das Injektionsgerät als Spritze ausgebildet sein. Das Injektionsgerät kann als separate Komponente der Vorrichtung vorliegen. Polyethylenglycol kann ggf. in Lösung mit einer beliebigen Anzahl weiterer Stoffe oder als Suspension mit einer beliebigen Anzahl weiterer Stoffe vorliegen.

Vorzugsweise enthält das Injektionsgerät eine Injektionsflüssigkeit, wobei die Injektionsflüssigkeit Polyethylenglycol enthält. Polyethylenglycol kann die Wirksamkeit einer Behandlung mit der Vorrichtung verbessern.

Beispielhaft offenbart, jedoch nicht als solches beansprucht, wird ein kaltes atmosphärisches Plasma als medizinischer Stoff im Rahmen einer weiteren medizinischen Indikation, nämlich der Anwendung in einem Verfahren zur Behandlung von Bandscheibenvorfällen. Es steht dabei vornehmlich die Behandlung chronischer Bandscheibenvorfälle in den Lenden- und Steißbeinwirbeln von Hunden aufgrund von fibroider Degeneration im Vordergrund. Dennoch sind auch Behandlungserfolge bei Katzen dokumentiert und es kann davon ausgegangen werden, dass die noch junge Forschung in diesem Gebiet zukünftig auch Indikationen zur Anwendung bei anderen Tierarten und im humanmedizinischen Bereich hervorbringen kann.

Beispielhaft offenbart, aber nicht als solches beansprucht, wird auch Polyethylenglycol zur Verwendung bei der Behandlung von Bandscheibenvorfällen, insbesondere unter zusätzlicher Anwendung von kaltem atmosphärischem Plasma. Insbesondere betrifft die Erfindung eine Polyethylenglycol enthaltende Injektionsflüssigkeit zur Verwendung bei der Behandlung von Bandscheibenvorfällen.

Weitere Vorteile der Erfindung ergeben sich aus der Beschreibung und der Zeichnung. Ebenso können die vorstehend genannten und die noch weiter ausgeführten Merkmale erfindungsgemäß jeweils einzeln für sich oder zu mehreren in beliebigen Kombinationen Verwendung finden. Die gezeigten und beschriebenen Ausführungsformen sind nicht als abschließende Aufzählung zu verstehen, sondern haben vielmehr beispielhaften Charakter für die Schilderung der Erfindung.

### Kurzbeschreibung der Zeichnungen

Es zeigen:
- Fig. 1: eine isometrische Ansicht einer Vorrichtung zur Behandlung von Bandscheibenvorfällen unter Erzeugung kalten atmosphärischen Plasmas sowie einen beispielhaften, nicht-maßstäblichen Patienten;
- Fig. 2a: eine isometrische Ansicht einer Explosionsdarstellung eines Handstücks der Vorrichtung zur Behandlung von Bandscheibenvorfällen;
- Fig. 2b: die Explosionsdarstellung aus Fig. 2a in einer Seitenansicht;
- Fig. 3a: eine isometrische Ansicht einer Dielektrikumsscheibe;
- Fig. 3b: einen Ausschnitt der Dielektrikumsscheibe sowie eine geschnittene Seitenansicht der Dielektrikumsscheibe.

### Detaillierte Beschreibung der Erfindung und Zeichnung

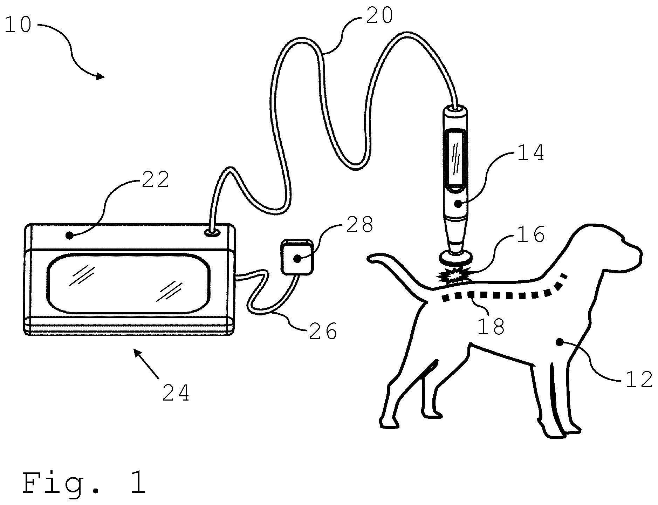

**Fig.** 1 zeigt eine isometrische Ansicht einer Vorrichtung **10** zur Behandlung von Bandscheibenvorfällen, insbesondere von chronischen Bandscheibenvorfällen bei Hunden **12** und Katzen. Der dargestellte Hund 12 dient der besseren Anschaulichkeit der Fig. 1 und ist nicht-maßstäblich abgebildet. Die Vorrichtung weist ein Handstück **14** auf, an dessen Ende kaltes atmosphärisches Plasma **16** erzeugt wird.

Das Handstück 14 wird dabei von einem Behandlungsexperten, insbesondere einem Veterinärmediziner, genutzt, um das Plasma 16 in einem Wirbelsäulenbereich **18** des Hundes 12, insbesondere im Bereich der Lenden- und Steißbeinwirbel, zu applizieren. Unterstützend kann vor, während und/oder nach der Applikation des Plasmas 16 eine Polyethylenglycol enthaltende Injektionsflüssigkeit in eine Umgebung des Wirbelsäulenbereichs 18, insbesondere an die Stelle, wo der Bandscheibenvorfall ist, injiziert werden (nicht im Detail dargestellt). Hierzu kann ein Injektionsgerät der Vorrichtung 10 verwendet werden.

Das Ende des Handstücks 14 wird äußerlich auf die Haut des Hundes 12 aufgesetzt. Das Handstück 14 ist über ein Kabel **20** an einer Kontrolleinheit **22** angeschlossen, in welcher ein Resonanzwandler **24** verbaut ist. Die Kontrolleinheit 22 ist über ein weiteres Kabel **26** mit einem Hausnetzanschluss **28** verbunden. Der Resonanzwandler 24 erzeugt unter Ausnutzung elektrischer Energie aus dem Hausnetzanschluss 28 hochfrequente elektrische Schwingungen, welche wiederum zur Erzeugung des Plasmas 16 zum Einsatz kommen.

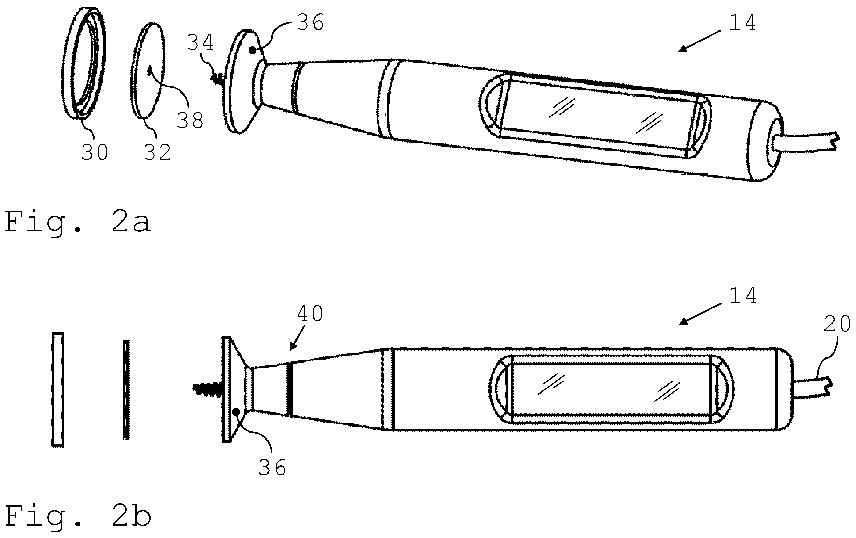

**Fig. 2a** zeigt eine isometrische Ansicht des Handstücks 14, wobei die Komponenten am plasmaerzeugenden Ende des Handstücks 14 im Rahmen einer Explosionsdarstellung abgebildet sind. Ein vorzugsweise aus Kunststoff bestehender Haltering **30** dient zum Einlegen einer Dielektrikumsscheibe **32.** Die Dielektrikumsscheibe 32 wird mithilfe einer Feder **34** an einem Kopfstück **36** des Handstücks 14 zwischen dem Haltering 30 und dem Kopfstück 36 verspannt. Der Haltering 30 kann vorzugsweise über eine Schraubverbindung (hier aus Gründen der Übersichtlichkeit nicht dargestellt) am Kopfstück 36 befestigt werden. Beim Zusammensetzen der Komponenten kann die Feder 34 in einen vorzugsweise kreisförmigen Eingriffsbereich **38** der Dielektrikumsscheibe 32 eingreifen.

**Fig. 2b** zeigt das Handstück 14 aus Fig. 2a in einer Seitenansicht. Das Kopfstück 36 des Handstücks 14 umschließt eine Elektrode **40** oder zumindest einen Teil der Elektrode 40. Die Elektrode 40 wird über das Kabel 20 mit elektrischer Hochfrequenzenergie versorgt. Das Kopfstück 36 besteht dabei aus einem elektrisch isolierenden Material, vorzugsweise Kunststoff.

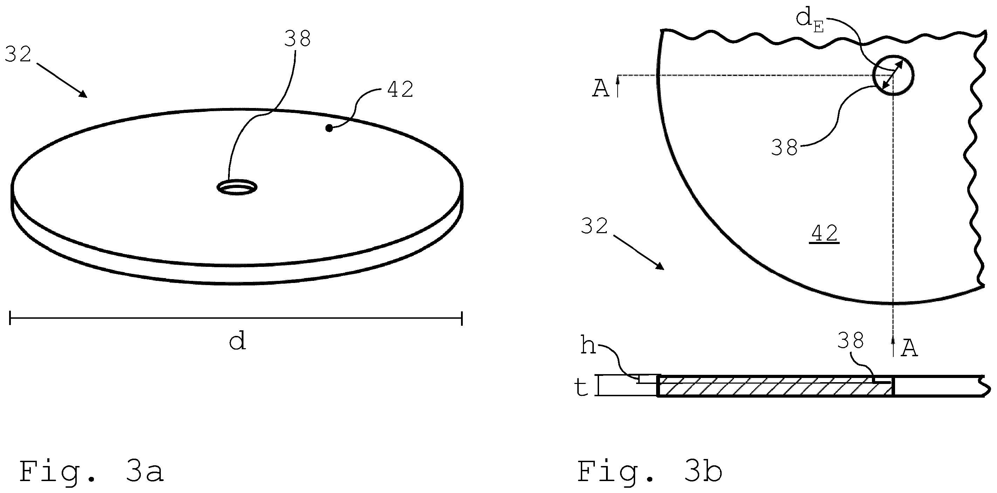

**Fig. 3a** zeigt die Dielektrikumsscheibe 32, wobei der kreisförmige Eingriffsbereich 38 erkennbar ist. Der Eingriffsbereich 38 befindet sich auf der Rückseite **42** der Dielektrikumsscheibe 32, d.h. auf der dem Kopfstück 36 zugewandten Seite. Die Dielektrikumsscheibe 32 weist einen Durchmesser d zwischen 14 mm und 51 mm auf.

**Fig. 3b** zeigt einen Ausschnitt der Dielektrikumsscheibe 32 sowie eine geschnittene Seitenansicht der Dielektrikumsscheibe 32 entlang der Schnittkante A-A. Der Eingriffsbereich 38 weist einen Durchmesser d_{E} zwischen 2 mm und 3 mm auf. Zusätzlich dazu weist der Eingriffsbereich 38 eine Höhe h von mindestens 0,1 mm und maximal 0,4 mm auf. Die Wandstärke t der Dielektrikumsscheibe 32 liegt zwischen minimal 1,2 mm und maximal 1,73 mm.

Unter Vornahme einer Zusammenschau aller Figuren der Zeichnung betrifft die Erfindung zusammenfassend eine Vorrichtung 10 zur Behandlung von Bandscheibenvorfällen, insbesondere im Veterinärwesen, vorzugsweise bei Hunden 12 und Katzen. Es wird ein Gerät zur Erzeugung kalten atmosphärischen Plasmas 16 mit einer Elektrode 40 und ein die Elektrode 40 zumindest teilweise umgebendes plattenförmiges Dielektrikum 32 genutzt. Die Erfindung ist dadurch gekennzeichnet, dass das Dielektrikum 32 zur Erzielung einer Tiefenwirkung im Wirbelsäulenbereich 18 eines behandelten Körpers ausgelegt ist. Dafür weist das Dielektrikum 32 eine Wandstärke t von mindestens 1,2 mm und maximal 1,73 mm auf. Die Erfindung betrifft weiterhin kaltes atmosphärisches Plasma 16 zur Anwendung in einem Verfahren zur Behandlung von Bandscheibenvorfällen.

### Bezugszeichenliste

- 10: Vorrichtung zur Behandlung von Bandscheibenvorfällen
- 12: Hund
- 14: Handstück
- 16: kaltes atmosphärisches Plasma
- 18: Wirbelsäulenbereich
- 20: Kabel
- 22: Kontrolleinheit
- 24: Resonanzwandler
- 26: weiteres Kabel
- 28: Hausnetzanschluss
- 30: Haltering
- 32: Dielektrikumsscheibe
- 34: Feder
- 36: Kopfstück
- 38: Eingriffsbereich
- 40: Elektrode
- 42: Rückseite

## Patentansprüche

1. Vorrichtung (10) zur Behandlung von Bandscheibenvorfällen, insbesondere im Veterinärwesen, vorzugsweise bei Hunden (12) und Katzen, wobei die Vorrichtung (10) ein Gerät zur Erzeugung kalten atmosphärischen Plasmas (16) mit einer Elektrode (40) und ein die Elektrode (40) zumindest teilweise umgebendes plattenförmiges Dielektrikum (32) aufweist, wobei das Dielektrikum (32) eine Wandstärke t von mindestens 1,2 mm und maximal 1,73 mm aufweist, um eine Tiefenwirkung im Wirbelsäulenbereich eines behandelten Körpers zu erzielen,
**dadurch gekennzeichnet, dass**
das Dielektrikum (32) einen Eingriffsbereich (38) mit einer Höhe h von mindestens 0,1 mm und maximal 0,4 mm aufweist.

2. Vorrichtung (10) nach Anspruch 1, bei der das Dielektrikum (32) Kunststoff, insbesondere ein Acrylnitril-Butadien-Styrol-Copolymer, aufweist.

3. Vorrichtung (10) nach Anspruch 1, bei der das Dielektrikum (32) einen keramischen Werkstoff aufweist.

4. Vorrichtung (10) nach einem der vorhergehenden Ansprüche, bei der das Dielektrikum (32) in Form einer flachen Scheibe vorliegt, welche insbesondere einen Durchmesser d zwischen 14 mm und 51 mm aufweist.

5. Vorrichtung (10) nach einem der vorhergehenden Ansprüche, bei der der Eingriffsbereich (38) des Dielektrikums (32) kreisrund ausgebildet ist, wobei er vorzugsweise einen Durchmesser d_{E} zwischen 2 mm und 3 mm aufweist.

6. Vorrichtung (10) nach einem der vorhergehenden Ansprüche, welche einen Resonanzwandler (24) aufweist, um die zur Plasmaerzeugung notwendige elektrische Hochfrequenz- oder Hochspannungsenergie über einen elektrischen Hausnetzanschluss (28) bereitzustellen.

7. Vorrichtung (10) nach einem der vorhergehenden Ansprüche, wobei die Vorrichtung (10) ein Injektionsgerät zur Injektion von Polyethylenglycol aufweist, insbesondere wobei das Injektionsgerät als Spritze ausgebildet ist.

8. Vorrichtung (10) nach Anspruch 7, wobei das Injektionsgerät eine Injektionsflüssigkeit enthält, wobei die Injektionsflüssigkeit Polyethylenglycol enthält.

## Claims

1. An apparatus (10) for treating intervertebral disc incidents, in particular in veterinary medicine, preferably in dogs (12) and cats, the apparatus (10) comprising a device for generating cold atmospheric plasma (16) with an electrode (40) and a plate-shaped dielectric (32) at least partially surrounding the electrode (40), the dielectric (32) having a wall thickness t of at least 1.2 mm and at most 1.73 mm so as to achieve a depth effect in the spinal region of a treated body, **characterized in that**
the dielectric (32) has an engagement region (38) with a height h of at least 0.1 mm and at most 0.4 mm.

2. The apparatus (10) according to claim 1, wherein the dielectric (32) comprises plastics material, in particular an acrylonitrile butadiene styrene copolymer.

3. The apparatus (10) according to claim 1, wherein the dielectric (32) comprises a ceramic material.

4. The apparatus (10) according to any of the preceding claims, wherein the dielectric (32) is in the form of a flat disc, which in particular has a diameter d between 14 mm and 51 mm.

5. The apparatus (10) according to any of the preceding claims, wherein the engagement region (38) of the dielectric (32) is circular, preferably having a diameter d_{E} between 2 mm and 3 mm.

6. The apparatus (10) according to any of the preceding claims, which has a resonant converter (24) so as to provide the electrical high frequency or high voltage energy necessary for plasma generation via a household electrical mains connection (28).

7. The apparatus (10) according to any of the preceding claims, wherein the apparatus (10) comprises an injection device for injecting polyethylene glycol, in particular wherein the injection device is designed as a syringe.

8. The apparatus (10) according to claim 7, wherein the injection device contains an injection fluid, wherein the injection fluid contains polyethylene glycol.

## Revendications

1. Dispositif (10) pour le traitement de l' hernie discale, en particulier en médecine vétérinaire, de préférence chez le chien (12) et le chat, le dispositif (10) comprenant un appareil de génération de plasma atmosphérique froid (16) avec une électrode (40) et un diélectrique en forme de plaque (32) entourant au moins partiellement l'électrode (40), le diélectrique (32) présentant une épaisseur de paroi t d'au moins 1,2 mm et au maximum 1,73 mm afin d'obtenir un effet de profondeur dans la région vertébrale d'un corps traité,
**caractérisé en ce que**
le diélectrique (32) présente une zone d'intervention (38) avec une hauteur h d'au moins 0,1 mm et au maximum 0,4 mm.

2. Dispositif (10) selon la revendication 1, dans lequel le diélectrique (32) comprend une matière plastique, en particulier un copolymère acrylonitrile-butadiène-styrène.

3. Dispositif (10) selon la revendication 1, dans lequel le diélectrique (32) comprend un matériau céramique.

4. Dispositif (10) selon l'une des revendications précédentes, dans lequel le diélectrique (32) se présente sous la forme d'un disque plat, lequel présente en particulier un diamètre d compris entre 14 mm et 51 mm.

5. Dispositif (10) selon l'une des revendications précédentes, dans lequel la zone d'intervention (38) du diélectrique (32) est de forme circulaire, présentant de préférence un diamètre d_{E} compris entre 2 mm et 3 mm.

6. Dispositif (10) selon l'une des revendications précédentes, comprenant un convertisseur de résonance (24) afin de fournir l'énergie électrique haute fréquence ou l'énergie électrique haute tension nécessaire à la génération de plasma via un raccordement au réseau électrique domestique (28).

7. Dispositif (10) selon l'une des revendications précédentes, le dispositif (10) comprenant un dispositif d'injection pour l'injection de polyéthylène glycol, en particulier le dispositif d'injection étant conçu comme une seringue.

8. Dispositif (10) selon la revendication 7, le dispositif d'injection contenant un liquide d'injection, le liquide d'injection contenant du polyéthylène glycol.
